# EUROPEAN PATENT SPECIFICATION

(11) **EP 1 516 622 B1**
(45) Date of publication and mention of the grant of the patent: **24.10.2007**
(21) Application number: 03398006.1
(22) Date of filing: 18.09.2003
(51) Int. Cl.: A61K 31/554, A61K 9/06, A61K 47/06, A61K 47/44, A61P 9/08

(54) **Stable diltiazem hydrochloride pharmaceutical composition for cutaneous application and process for the preparation thereof**
Stabile Diltiazemhydrochlorid enthaltende pharmazeutische Zusammensetzung zur Anwendung auf der Haut und Verfahren zu ihrer Herstellung
Composition pharmaceutique stable de chlorhydrate de diltiazem pour application cutanée et procédé pour sa préparation

(43) Date of publication of application: 23.03.2005
(73) Proprietor: TECNIMEDE-SOCIEDADE TECNICO-MEDICINAL, S.A., 2685-338 Prior Velho (PT)
(72) Inventor: Velga Abreu Rocha, Alexandre Miguel, 3050-208 Luso (PT); Castro de Abreu, Isabel Maria, 2670-386 Loures (PT)
(74) Representative: Ferreira, Maria Silvina

(56) References cited:
- WO-A-02/49603
- WO-A-92/03121
- US-A- 5 019 395
- MUSZALSKA IZABELA ET AL: "Kinetics of diltiazem hydrochloride in solid phase." ACTA POLONIAE PHARMACEUTICA. POLAND 2003 JAN-FEB, vol. 60, no. 1, January 2003 (2003-01), pages 27-30, XP009025725 ISSN: 0001-6837
- ANDRISANO V ET AL: "Photostability and phototoxicity studies on diltiazem" JOURNAL OF PHARMACEUTICAL AND BIOMEDICAL ANALYSIS, vol. 25, no. 3-4, June 2001 (2001-06), pages 589-597, XP002270135 ISSN: 0731-7085

## Description

### FIELD OF THE INVENTION

The present invention concerns a stable diltiazem hydrochloride pharmaceutical composition for cutaneous application, comprising a content of deacethyldiltiazem of less than 0.3 % (w/w), as well as a process for the preparation thereof.

### BACKGROUND OF THE INVENTION

Diltiazem hydrochloride is a calcium channel blocker. An increase in the cytoplasmatic concentration of Ca²⁺ ions causes the rise of the myocardial contraction and of the vascular smooth muscle. The uptake of extracellular Ca²⁺ is more important for the initiation of the contraction of myocardial cells, whereas the release of Ca²⁺ from the intracellular storage sites also participates in the vascular smooth muscle (Goodman and Gilman's, 8^{th} edition, 1990).

The *in vitro* degradation of diltiazem hydrochloride is potentiated in acidic medium and at high temperatures. The main degradation product is deacethyldiltiazem (Martindale The Complete Drug Reference, 32^{nd} edition, The Pharmaceutical Press). This degradation product (deacethyldiltiazem) has about 50% of the potency of diltiazem hydrochloride as a vasodilator (Goodman and Gilman's, 8^{th} edition, 1990).

European Patent EP 0 275 054, filed on January 9, 1998, refers as example the composition and preparation process for the formulation of a 1% diltiazem hydrochloride rectal infusion. This rectal infusion comprises in its composition a carboxyvinyl polymer and an amino acid (L-valine). According to EP 0 275 054 this formulation, which contains water in its composition, has a pH value of 6.9.

International Patent application WO 98/36733, filed on February 23, 1998, describes a pharmaceutical composition for cutaneous application, which comprises at least one cholinergic agent or a calcium channel blocker, for local anal administration, for the treatment of benign anal disorders. This patent application refers as formulation examples, a formulation in the form of a gel which comprises sodium carmelose and polyethylene glycol, and a formulation in the form of an emulgel which comprises carbomer, propylene glycol, dimethyl sulphoxide and oily excipients. Both formulations have water in its composition.

European Patent EP 0 526 561, filed on April 16, 1991, discloses a composition for transdermic administration of drugs. In this patent, a formulation containing an ethanol aqueous solution and a slight exciding amount of Octanol is described.

The diltiazem hydrochloride is used as a vasodilator for cutaneous application. However, the formulations which are part of the state of the art are unstable with the formation of the degradation product deacethyldiltiazem. As mentioned above, this product has only 50% of the vasodilator capacity of diltiazem hydrochloride, causing thereby a decrease in the vasodilator activity of the formulations for cutaneous application.

### SUMMARY OF THE INVENTION

It is object of the present invention a stable diltiazem hydrochloride pharmaceutical composition for cutaneous application, comprising a deacethyldiltiazem content of less than 0.3% (w/w) in the final product, as well as a process for the preparation thereof.

The pharmaceutical composition according to the invention consists in a diltiazem hydrochloride paste, comprising in its composition only oily excipients.

The diltiazem hydrochloride paste that has a deacethyldiltiazem content of less than 0.3% (w/w) and which is object of the present invention is obtained by the melting of the oily components, in which the diltiazem hydrochloride is subsequently dispersed. According to the present invention, the diltiazem hydrochloride is dispersed at a specific temperature, which allows to obtain a homogeneous active substance dispersion, but does not promote the formation of deacethyldiltiazem.

The qualitative and quantitative composition and the preparation process according to the present invention allows to obtain a stable formulation during shelf life, with good properties for application to the skin and mucosa, and with excellent vasodilator properties, due to its low deacethyldiltiazem content (< 0.3%).

### DETAILED DESCRIPTION OF THE INVENTION

It is an object of the present invention the obtention of a stable pharmaceutical composition, suitable for cutaneous application and with a deacethyldiltiazem content of less than 0.3% (w/w). This is achieved by the combination of diltiazem hydrochloride to oily excipients, to one or more tensioactive agents, and one or more preservatives, in the absence of water or any other aqueous excipient.

The diltiazem hydrochloride can be included in the formulation according to the invention in concentrations between 0.5% and 10% of the formulation total mass, preferentially between 2% and 6% of the formulation total mass.

The oily excipients may be selected from the oily excipients generally used in the semi-solid pharmaceutical forms, provided that its association allows the obtention of a formulation having suitable characteristics for cutaneous application. For example, as oily excipients, emollients (Cetiol LC®, solid and/or liquid paraffin, vegetal oils), thickeners (ketostearyl alcohol) may be used.

The tensioactive agents may be selected among those that act simultaneously as tensioactives and as cutaneous absorption promoters. Examples of tensioactive agents, acting simultaneously as cutaneous absorption promoters, are polysorbate 40, polysorbate 80.

The elected preservative should be soluble in one of the selected oily excipients. As preservatives, benzoic acid, sorbic acid, phenylethyl alcohol and chorbutanol may be used.

The formulation pH value should range between 2.5 and 5, preferably between 3.5 and 4.5.

The formulation final viscosity should range between 100 mPas and 1500 mPas, preferably 200 mPas and 500 mPas.

According to one way of preparation, the preservative is dissolved in one of the oily components of the formulation. The solid oily excipients are melted at a temperature which is higher than their melting point. The temperature is decreased to 40°C to 65°C, or preferably to 45°C to 55°C, and the active substance is homogenously dispersed. It is cooled down to room temperature, under stirring.

The present invention is illustrated by the following examples and assays.

### EXAMPLE 1: 2% (w/w) Diltiazem hydrochloride aqueous solution

### 1 - Composition

**TABLE 1: Qualitative and quantitative composition of the 2% (w/w) diltiazem hydrochloride solution**

| **Composition (%) / lot** | ***DILC23- 020161A*** |
|---|---|
| **Diltiazem hydrochloride** | 2.0 |
| **Water** | 98.0 |
| **TOTAL** | 100.0 |

### 2 - Preparation

The diltiazem hydrochloride was dissolved in water.
The obtained solution was filled into amber glass flasks.

### 3 - Assessment of the solution stability

The flasks containing the solution were stored at 25°C/60% RH and at 50°C. The solution was analysed for day 0, 2, 7 and 12. The performed tests consisted in the assessment of the aspect, pH determination, assay and related compounds (in accordance with the European Pharmacopoeia monograph, 4th edition, 2002, for diltiazem hydrochloride).

The results are shown in tables 4 to 6.

### EXAMPLE 2: 2% (w/w) diltiazem hydrochloride aqueous solution, containing sodium benzoate

### 1 - Composition

**TABLE 2: Qualitative and quantitative composition of the 2% (w/w) diltiazem hydrochloride solution**

| **Composition (%) / lot** | ***DILC23- 020161B*** |
|---|---|
| **Diltiazem Hydrochloride** | 2.0 |
| **Water** | 97.8 |
| **Sodium Benzoate** | 0.2 |
| **TOTAL** | 100.0 |

### 2 - Preparation

The sodium benzoate was dissolved in water. The diltiazem hydrochloride was dissolved in the sodium benzoate solution.

The obtained solution was filled into amber glass flasks.

### 3 - Assessment of the solution stability

The flasks containing the solution were stored at 25°C/60% RH and at 50°C. The solution was analysed for day 0, 2, 7 and 12. The performed tests consisted in the assessment of the aspect, pH determination, assay and related compounds (in accordance with the European Pharmacopoeia monograph, 4th edition, 2002, for diltiazem hydrochloride).

The results are shown in tables 4 to 6.

### EXAMPLE 3: 2% (w/w) diltiazem hydrochloride aqueous solution, containing propylene glycol

### 1 - Composition

**TABLE 3: Qualitative and quantitative composition of the 2% (w/w) diltiazem hydrochloride solution**

| **Composition (%) / lot** | ***DL6-01006-* 1*C*** |
|---|---|
| **Diltiazem Hydrochloride** | 2.0 |
| **Water** | 5.0 |
| **Propylene glycol** | 93.0 |
| **TOTAL** | 100.0 |

### 2 -Preparation

The diltiazem hydrochloride was dissolved in water. The propylene glycol was added to the solution.

The obtained solution was filled into amber glass flasks.

### 3 - Assessment of the solution stability

The flasks containing the solution were stored at 25°C/60% RH and at 50°C. The solution was analysed for day 0, 2, 7 and 12. The performed tests consisted in the assessment of the aspect, pH determination, assay and related compounds (in accordance with the European Pharmacopoeia monograph, 4th edition, 2002, for diltiazem hydrochloride).

The results are shown in tables 4 to 6.

Tables 4 to 6 show the results (aspect, pH, assay and related compounds) of the stability study performed for the solution referred in EXAMPLE 1, EXAMPLE 2 and EXAMPLE 3.

The solutions obtained were clear and colourless. The aspect did not suffer any changes during the storage period.

**TABLE 4: pH Values for the diltiazem hydrochloride solutions**

| **Lot / Storage Conditions** | **pH** | | | | | |
|---|---|---|---|---|---|---|
| | **0 d** | **2 d** | **7 d** | | **12 d** | |
| | **25°C/60% RH** | **50°C** | **25°C/60% RH** | **50°C** | **25°C/60% RH** | **50°C** |
| ***DILC23- 020161A*** | 4.61 | 4.30 | 4.72 | 3.94 | 4.74 | 3.80 |
| ***DILC23- 020161B*** | 5.64 | 5.50 | 5.40 | 4.84 | 5.36 | 4.76 |
| ***DL6-01006- 1C*** | 4.27 | n.p. | n.p. | 4.19 | n.p. | n.p. |
| **d -** days; **n.p. -** not performed | | | | | | |

**TABLE 5: Assay of diltiazem hydrochloride in the solutions**

| **Lot / Storage Conditions** | ⁽²⁾**Assay ± V.C. (%)** | | | | | |
|---|---|---|---|---|---|---|
| | **0 d** | **2 d** | **7 d** | | **12 d** | |
| | **25°C/60% RH** | **50°C** | **25°C/60% RH** | **50°C** | **25°C/60% RH** | **50°C** |
| ***DILC23-020161A*** | 98.86 ± 0.11 | 99.32 ± 0.60 | 98.78 ± 1.59 | 95.00 ± 0.28 | n.p. | n.p. |
| ***DILC23-020161B*** | 102.29 ± 1.19 | 95.84 ± 1.26 | 97.32 ± 0.06 | 96.76 ± 0.07 | n.p. | n.p. |
| ***DL6-01006-1C*** | 100.95 ± 0.22 | 101.00 ± 0.26 | n.p | 99.86 ± 0.33 | 97.70 ± 0.048 | 89.79 ± 0.99 |

| | | | | | | |
|---|---|---|---|---|---|---|
| **V.C.** - Variation Coefficient; **d** - days; **n.p.** - not performed; ⁽²⁾-mean of 2 samples | | | | | | |

**TABLE 6: Assay of the related compounds in the diltiazem hydrochloride solutions**

| **Lot / Storage Conditions** | **Related Compounds (%)** | | | | | |
|---|---|---|---|---|---|---|
| | **0 d** | **2 d** | **7 d** | | **12 d** | |
| | **25°C/60 % RH** | **50°C** | **25°C/60 % RH** | **50°C** | **25°C/60 % RH** | **50°C** |
| ***DILC23-020161A*** | 0.055⁽¹⁾ | 0.527⁽¹⁾ | 0.238⁽¹⁾ | 1.314⁽¹⁾ | n.p. | n.p. |
| ***DILC23-020161B*** | 0-184⁽¹⁾ | 1.837⁽¹⁾ 0.023 | 1.032⁽¹⁾ | 5.339⁽¹⁾ | n.p. | n.p. |
| ***DL6-01006-1C*** | 0.048⁽¹⁾ 0.022 | 0.079⁽¹⁾ 0.020 | n.p. | 0.280⁽¹⁾ | 0.106⁽¹⁾ | 0.428⁽¹⁾ |
| **d** - days; **n.p**. - not performed; ⁽¹⁾ Deacethyldiltiazem | | | | | | |

In accordance with the performed studies it was possible to conclude that the diltiazem hydrochloride is unstable in aqueous medium. This active substance is hydrolysed in acidic or alkaline medium, with the production of deacethyldiltiazem, which has about 50% of the vasodilator capacity of diltiazem hydrochloride.

### EXAMPLE 4: 1.0% (w/w) Diltiazem hydrochloride aqueous gel

The following example is part of the state of the art (European Patent EP 275054) and will be used for comparison with the formulation, object of the present invention.

### 1 - Composition

**TABLE 7 : Qualitative and quantitative composition of the 1% (w/w) diltiazem hydrochloride gel**

| **Composition (%) / lot** | ***DILC22-020181E*** |
|---|---|
| **Diltiazem hydrochloride** | 1.0 |
| **4% (w/w) Carbopol solution** | 12.5 |
| **4% (w/w) L-valine aqueous solution** | 25.0 |
| **2% (w/v) Sodium hydroxide aqueous solution** | 10.0 |
| **Purified water** | 51.5 |
| **TOTAL** | 100.0 |

### 2 - Preparation

Purified water is added to the carboxyvinyl polymer aqueous solution. Subsequently, the diltiazem is gradually added under stirring. The L-valine solution is added very slowly to this mixture and the resulting mixture is uniformly stirred in order to increase the viscosity. Finally, the sodium hydroxide solution is added. The mixture is stirred so that a gel is obtained.

The gel was packed in amber glass flasks.

### 3 - Assessment of the stability

The flasks containing the formulation were stored at 25°C/60%RH and at 50°C, analyses having been performed for day 0, day 2 and day 8. The formulation was characterized with respect to its aspect, pH, viscosity, assay and related compounds (in accordance with the European Pharmacopoeia monograph, 4th edition, 2002, for diltiazem hydrochloride).

The results are shown in Tables 12 to 16.

### EXAMPLE 5: 2.0% (w/w) Diltiazem hydrochloride aqueous gel

This example corresponds to an example referred in the European Patent application EP 969813 and will be used for comparison with the formulation, object of the present invention.

### 1 - Composition

**TABLE 8: Qualitative and quantitative composition of the 2% (w/w) diltiazem hydrochloride gel**

| **Composition / lot** | ***DILC22-020181F*** |
|---|---|
| **Diltiazem hydrochloride** | 2.0 g |
| **Sodium Carmelose** | 6.0 g |
| **Polyethylene glycol** | 30.0 ml |
| **Methyl p-hydroxybenzoate** | 150.0 mg |
| **Propyl p-hydroxybenzoate** | 15.0 mg |
| **Purified water (to complete)** | s.q.t. |
| **TOTAL** | 100.0 g |
| **s.q.t** - sufficient quantity to | |

### 2 - Preparation

The preservatives are dissolved in part of the water (80°C). It is cooled down to room temperature. The diltiazem hydrochloride is added to the obtained solution and stirred until complete dissolution. Following the obtention of a solution, the polyethylene glycol and the sodium carmelose are added. The remaining water is added to the obtained gel.

The gel was packed in amber glass flasks.

### 3 - Assessment of the stability

The flasks containing the gel were stored at 25°C/60%RH and 50°C. Analyses were performed for day 0, day 2 and day 8. The formulation was characterized with respect to its aspect, pH, viscosity, assay and related compounds (in accordance with the European Pharmacopoeia monograph, 4th edition, 2002, for diltiazem hydrochloride).

The results are shown in Tables 12 to 16.

### EXAMPLE 6: 2.0% (w/w) diltiazem hydrochloride aqueous Emulgel

This example was referred in the European Patent application EP 969813 and will be used for comparison with the formulation, object of the present invention.

### 1 - Composition

**TABLE 9 : Qualitative and quantitative composition of the 2% (w/w) diltiazem hydrochloride emulgel**

| **Composition (g) / lot** | ***DILC21-020181B*** |
|---|---|
| **Diltiazem hydrochloride** | 10.0 |
| **Dimethyl sulphoxide** | 250.0 |
| **Carbomer** | 5.0 |
| **White solid Paraffin** | 15.0 |
| **Ketomacrogel** | 115.0 |
| **Propylene glycol** | 23.0 |
| **Methyl p-hydroxybenzoate solution** | s.q.t. |
| **TOTAL** | 500 |
| **s.q.t** - sufficient quantity to | |

### 2 -Preparation

The propylene glycol is added to the preservative aqueous solution. The carbomer is dispersed in the obtained solution, so that a colloidal suspension is obtained. Then, the dimethyl sulphoxide is added under strong agitation, so that a translucent gel is obtained.

On the other hand, the oily phase components are melted.

The diltiazem hydrochloride is dissolved in the remaining preservative solution. The diltiazem hydrochloride solution is then added to the gel. Finally, the oily phase components are added to the gel.

The emulgel was packed in amber glass flasks.

### 3 - Assessment of the stability

The flasks containing the emulgel were stored at 25°C/60%RH and 50°C, analyses having been performed for day 0, day 2, day 8 and day 90. The formulation was characterized with respect to its aspect, pH, viscosity, assay and related compounds (in accordance with the European Pharmacopoeia monograph, 4th edition, 2002, for diltiazem hydrochloride).

The results are shown in Tables 12 to 16.

### EXAMPLE 7: Diltiazem hydrochloride hydrogel

The following example corresponds to an example referred in the European Patent EP 0 526 561 and will be used for comparison with the formulation, object of the present invention.

### 1 - Composition

**TABLE 10: Qualitative and quantitative composition of the diltiazem hydrochloride hydrogel**

| **Composition / lot** | ***DILC22-020181G*** |
|---|---|
| **Diltiazem hydrochloride** | 902.0 mg |
| **25% (v/v) Ethanol aqueous solution** | 10.0 ml |
| **Octanol** | 100.0 ml |
| **Polyvinyl pirrolidone** | 1000.0 mg |

### 2 - Preparation

The diltiazem hydrochloride is dissolved in the ethanol aqueous solution. The octanol is added and afterwards the polyvinyl pirrolidone is dissolved.

The hydrogel was packed in amber glass flasks.

### 3 - Assessment of the stability

The flasks containing the hydrogel were stored at 25°C/60%RH and at 50°C, analyses having been performed for day 0, day 2 and day 8. The formulation was characterized with respect to its aspect, pH, viscosity, assay and related compounds (in accordance with the European Pharmacopoeia monograph, 4th edition, 2002, for diltiazem hydrochloride).

The results are shown in Tables 12 to 16.

### EXAMPLE 8: 6.0% (w/w) diltiazem hydrochloride paste

### 1 - Composition

**TABLE 11: Qualitative and quantitative composition of the 6.0% (w/w) diltiazem hydrochloride paste**

| **Composition (%) / lot** | ***DILC23-020181A*** |
|---|---|
| **Diltiazem hydrochloride** | 6.00 |
| **Cetiol LC^{®} (coco-caprilate/caprate)** | 52.80 |
| **Liquid Paraffin** | 12.00 |
| **Tween 40^{®} (polysorbate 40)** | 10.00 |
| **Lanette O^{®} (Ketostearyl alcohol)** | 19.00 |
| **Benzoic acid** | 0.20 |

### 2 - Preparation

Briefly, in a container of proper dimensions, the Lanette O® (thickener), the liquid paraffin (emollient/oily carrier) and the Tween 40® (tensioactive/cutaneous absorption promoter) are melted at 70°C. After the obtention of a homogeneous phase the temperature is decreased to 50°C. The o Cetiol LC® (emollient) and the benzoic acid (preservative) are then added to oily phase obtained, under stirring. After obtaining a homogenous oily phase, the diltiazem hydrochloride (active substance) is dispersed under strong agitation.

The paste was packed in phenol coated aluminium tubes.

### 3 - Assessment of the paste stability

The tubes containing the paste were stored at 25°C/60%RH and at 50°C, analyses having been performed for day 0, day 2, day 8 and day 90. The formulation was characterized with respect to its aspect, pH, viscosity, assay and related compounds (in accordance with the European Pharmacopoeia monograph, 4th edition, 2002, for diltiazem hydrochloride).

The results are shown in Tables 12 to 16.

Tables 12 to 16 show the results obtained from the stability study performed (aspect, pH, viscosity, assay and related compounds) for the formulations referred in EXAMPLE 4, EXAMPLE 5, EXAMPLE 6, EXAMPLE 7 and EXAMPLE 8.

**TABLE 12: Aspect of the diltiazem hydrochloride formulations**

| **Lot / Storage conditions** | **Aspect** | | | | | |
|---|---|---|---|---|---|---|
| | **0 d** | **2 d** | **8 d** | | **90 d** | |
| | **25°C/60%RH** | **50°C** | **25°C/60%RH** | **50°C** | **25°C/60%RH** | **50°C** |
| ***DILC22-020181E*** | White aqueous Gel | | | | n.p. | |
| ***DILC22-020181F*** | Aqueous, viscous, translucent Gel | | | | n.p. | |
| ***DILC21-020181B*** | White aqueous Emulgel | | | | White aqueous Emulgel | n.p. |
| ***DILC22-020181G*** | Whitish hydrogel | | | | n.p. | |
| ***DILC23-020181A*** | Bright white Paste | Bright white Paste with diltiazem hydrochloride sedimentation | Bright white Paste | Bright white Paste with diltiazem hydrochloride sedimentation | Bright white Paste | n.p. |
| **d** - days; **n.p**. - not performed | | | | | | |

**TABLE 13: pH Values for the diltiazem hydrochloride formulations**

| **Lot / Storage conditions** | **pH** | | | | | |
|---|---|---|---|---|---|---|
| | **0 d** | **2 d** | **8 d** | | **90 d** | |
| | **25°C/60% RH** | **50°C** | **25°C/60%RH** | **50°C** | **25°C/60%RH** | **50°C** |
| ***DILC22-020181E*** | 6.77 | 6.51 | 6.52 | 6.07 | n.p. | |
| ***DILC22-020181F*** | 6.15 | 5.87 | 5.79 | 5.44 | n.p. | |
| ***DILC21-020181B*** | 4.45 | 4.48 | 4.86 | 4.56 | 3.36 | n.p |
| ***DILC22-020181G*** | 3.55 | 3.60 | 3.30 | 3.65 | n.p. | |
| ***DILC23-020181A*** | 3.15 | 3.12 | 3.13 | 3.10 | 2.99 | n.p |
| **d -** days; **n.p. -** not performed | | | | | | |

**TABLE 14: Viscosity Values for the diltiazem hydrochloride formulations**

| **Lot / Storage conditions** | **Viscosity (mPas)** | | | |
|---|---|---|---|---|
| | **0 d** | **2 d** | **8 d** | |
| | **25°C/60%RH** | **50°C** | **25°C/60%RH** | **50°C** |
| ***DILC22-020181E*** | 54.14 | n.p. | 84.39 | 17.39 |
| ***DILC22-020181F*** | 3255.21 | n.p. | 3205.60 | 2211.34 |
| ***DILC2I-020181B*** | 1777.11 | n.p. | 1909.08 | 1717.22 |
| ***DILC22-020181G*** | 9.34 | n.p. | 8.57 | 7.72 |
| ***DILC23-020181A*** | 228.73 | n.p. | 256.11 | 276.72 |
| **d** - days; **n.p.** - not performed | | | | |

**TABLE 15: Assay of diltiazem hydrochloride in the formulations**

| **Lot / Storage conditions** | ⁽²⁾**Assay ± V.C. (%)** | | | | | |
|---|---|---|---|---|---|---|
| | **0d** | **2d** | **8 d** | | **90 d** | |
| | **25°C/ 60% RH** | **50°C** | **25°C/60%RH** | **50°C** | **25°C/60%RH** | **50°C** |
| ***DILC22-020181E*** | 94.05 ± 0.96 | 79.44 ± 0.04 | 92.72 ± 1.52 | n.p. | n.p. | n.p. |
| ***DILC22-020181F*** | 96.49 ± 0.08 | 67.76 ± 0.04 | 91.70 ± 1.12 | n.p. | n.p. | n.p. |
| ***DILC21-020181B*** | 98.03 ± 0.08 | 98.32 ± 0.05 | n.p. | 99.61 ± 0.77 | 96.49 ± 0.07 | n.p. |
| ***DILC22-020181G*** | 97.20 ± 0.58 | 97.24 ± 0.09 | n.p. | 97.05 ± 0.69 | n.p. | n.p. |
| ***DILC23-020181A*** | 98.55 ± 0.89 | n.p. | 100.99 ± 0.13 | 98.87 ± 1.45 | 97.92 ± 0.87 | n.p. |
| **V.C.** - Variation coefficient; **d** - days; **n.p.** - not performed; ⁽²⁾ - mean of 2 samples | | | | | | |

**TABLE 16: Assay of the related compounds in the diltiazem hydrochloride formulations**

| **Lot / Storage conditions** | **Related compounds (%)** | | | | | |
|---|---|---|---|---|---|---|
| | **0 d** | **2 d** | **8 d** | | **90 d** | |
| | **25°C/60% RH** | **50°C** | **25°C/60% RH** | **50°C** | **25°C/60% RH** | **50° C** |
| ***DILC22-020181E*** | ⁽¹⁾1.783 | ⁽¹⁾15.803 | ⁽¹⁾7.651 | n.p. | n.p. | n.p. |
| ***DILC22-020181F*** | ⁽¹⁾0.882 | ⁽¹⁾2.470 | ⁽¹⁾2.729 | n.p. | n.p. | n.p. |
| ***DILC21-020181B*** | ⁽¹⁾0.046 | ⁽¹⁾0.194 | n.p. | ⁽¹⁾1.123 | 1.018 | n.p . |
| ***DILC22-020181G*** | ⁽¹⁾0.044 | ⁽¹⁾0.319 | n.p. | ⁽¹⁾ 2.202 | n.p.. | n.p. |
| ***DILC23-020181A*** | ⁽¹⁾0.052 | n.p. | ⁽¹⁾ 0.045 | ⁽¹⁾ 0.067 | ⁽¹⁾0.047 0.246 | n.p . |

| | | | | | | |
|---|---|---|---|---|---|---|
| **d -** days; **n.p. -** not performed; (1) deacethyldiltiazem | | | | | | |

Unexpectedly, it was ascertained that the deacethyldiltiazem content greatly increased for the formulations belonging to the state of the art (DILC22-020181E, DILC22-020181F, DILC21-020181B, DILC22-020181G), and consequently the diltiazem hydrochloride content decreased. The deacethyldiltiazem increase was verified even for those formulations stored at 25°C/60%RH, during a period of time of only 48 hours.

Even more unexpected was the fact that the deacethyldiltiazem content of the formulation according to the present invention (DILC23-020181A) was maintained under 0.3%, even after 90 days at a temperature of 25°C.

### EXAMPLE 9: 6.0% (w/w) Diltiazem hydrochloride Paste

The composition and preparation are identical to those described for EXAMPLE 8. This formulation corresponds to the lot number DL6-01006-1V.

### 1 - Assessment of the paste stability

The tubes containing the paste were stored at 25°C/60% RH and at 75°C. The paste was analysed for day 0, day 3 and day 7. The performed tests consisted in aspect assessment, pH determination, assay and related compounds (in accordance with the European Pharmacopoeia monograph, 4th edition, 2002, for diltiazem hydrochloride).

Tables 17 to 21 show the results (aspect, pH, assay and related compounds) of the accelerated stability study performed for the diltiazem hydrochloride paste of EXAMPLE 9.

**TABLE 17: Aspect of the diltiazem hydrochloride paste**

| **Time (days)** | **Storage conditions** | |
|---|---|---|
| | **25°C/60%RH** | **75°C** |
| **0** | Bright white paste | -- |
| **3** | -- | Bright white paste, with diltiazem hydrochloride sedimentation |
| **7** | Bright white paste | |

Table 17 describes the aspect of the paste stored at 25°C/60%RH and at 75°C. As it can be ascertained, the sedimentation of the diltiazem hydrochloride takes place at 75°C, due to the melting of the oily excipients. After homogenization during the cooling period, the paste reacquires a bright white colour.

**TABLE 18: pH Variation of the diltiazem hydrochloride paste**

| **Time (days)** | **Storage conditions** | |
|---|---|---|
| | **25°C/60%RH** | **75°C** |
| **0** | 3.16 | -- |
| **3** | -- | 3.01 |
| **7** | -- | 3.17 |

The pH of the 6% (w/w) diltiazem hydrochloride paste remained unchanged during the accelerated stability study, even after 7 days at 75°C.

**TABLE 19: Assay of the diltiazem hydrochloride**

| **Time (days)** | **Storage conditions** | |
|---|---|---|
| | **25°C/60%RH** | **75°C** |
| **0** | 100.26 ± 0.360 | -- |
| **3** | -- | 95.72 ± 2.327 |
| **7** | -- | 100.78 ± 1.128 |

**TABLE 20: Assay of benzoic acid**

| **Time (days)** | **Storage conditions** | |
|---|---|---|
| | **25°C/60%RH** | **75°C** |
| **0** | 96.52 ± 0.416 | -- |
| **3** | -- | 95.72 ± 2.327 |
| **7** | -- | 96.74 ± 0.318 |

**TABLE 21: Assay of the diltiazem hydrochloride related compounds**

| **Time (days))** | **Storage conditions** | |
|---|---|---|
| | **25°C/60%RH** | **75°C** |
| **0** | rrt 0.49 (Ddilc) = n.q. **Total** = **n.q.** | -- |
| **7** | -- | rrt 0.52 (Ddilc) = 0.231 rrt 1.82 = 0.026 **Total = 0.257** |
| **Ddilc** - Deacethyldiltiazem; **n.q.**- not quantifiable; **rrt** - relative retention time; Quantification Limit: Q.L. = 0.052% | | |

Surprisingly, the assay of the diltiazem hydrochloride and of the preservative benzoic acid remains stable, even after 7 days at 75°C (Tables 19 and 20). The related compounds (Table 21) suffered a light increase after 7 days at 75°C, but are maintained after this period of time, according to the specification for the final product (≤ 0.3%).

### EXAPLE 10: 6.0% (w/w) Diltiazem hydrochloride paste

The composition and preparation are identical to those described for EXAMPLE 8. This example corresponds to formulations which have the lot numbers DL6-01006-2A, DL6-01006-2B and DL6-01006-2C.

### 1 - Assessment of the paste stability

The tubes containing the paste were stored at 25°C/60%RH, 30°C/60%RH and 40°C/75%RH. The paste was analysed for 0 days, 3 months and 6 months. The tests performed consisted in the aspect assessment, pH determination, assay and related compounds (in accordance with the European Pharmacopoeia monograph, 4th edition, 2002, for diltiazem hydrochloride).

The aspect of the paste remains unchanged during the storage period.

Figures 1 to 12 show the results (pH, assay and related compounds) of the stability study performed for the diltiazem hydrochloride paste referred in EXAMPLE 10.
FIGURE 1: pH Variation during the stability (6 months, 25°C/60%RH);
FIGURE 2: Variation of the assay of diltiazem hydrochloride during the stability (6 months, 25°C/60%RH);
FIGURE 3: Variation of the benzoic acid assay during the stability (6 months, 25°C/60%RH);
FIGURE 4: Related compounds variation (6 months, 25°C/60%RH);
FIGURE 5: pH Variation during the stability (6 months, 30°C/60%RH);
FIGURE 6: Variation of the assay of diltiazem hydrochloride during the stability (6 months, 30°C/60%RH);
FIGURE 7: Variation of the benzoic acid assay during the stability (6 months, 30°C/60%RH);
FIGURE 8: Related compounds variation during the stability (6 months, 30°C/60%RH);
FIGURE 9: pH Variation during the stability (6 months, 40°C/75%RH);
FIGURE 10: Variation of the assay of diltiazem hydrochloride during the stability (6 months, 40°C/75%RH);
FIGURE 11: Variation of the benzoic acid assay during the stability (6 months, 40°C/75%RH);
FIGURE 12: Related compounds variation during the stability (6 months, 40°C/75%RH).

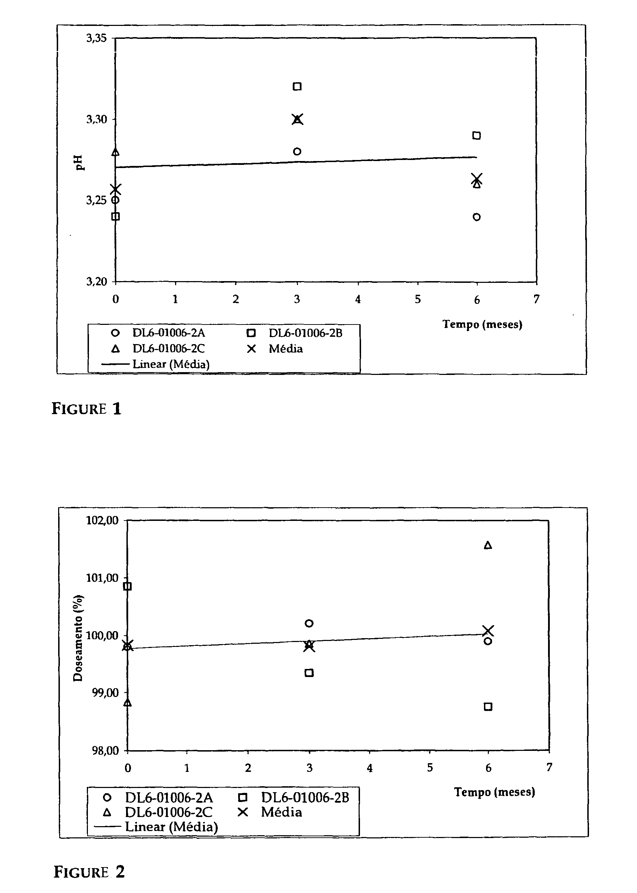

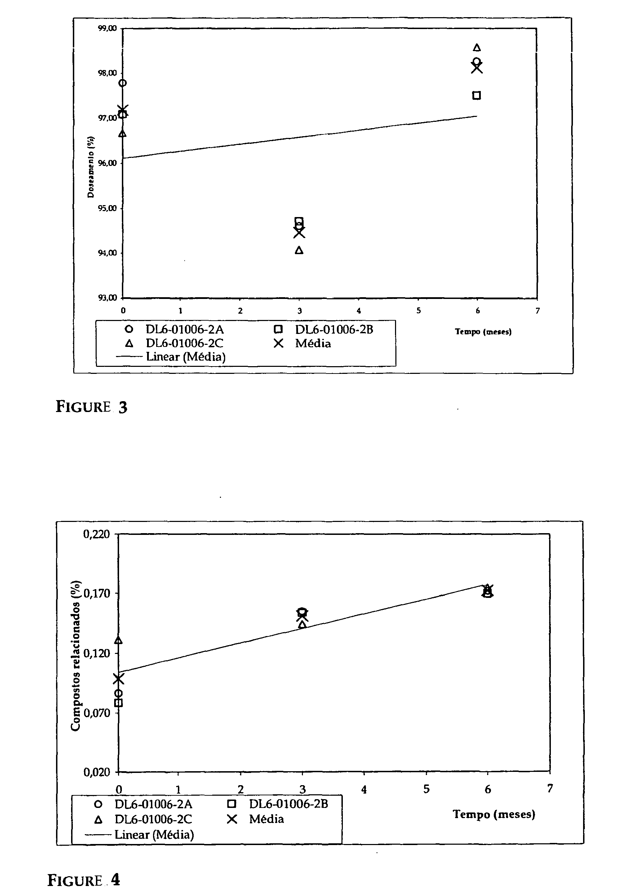

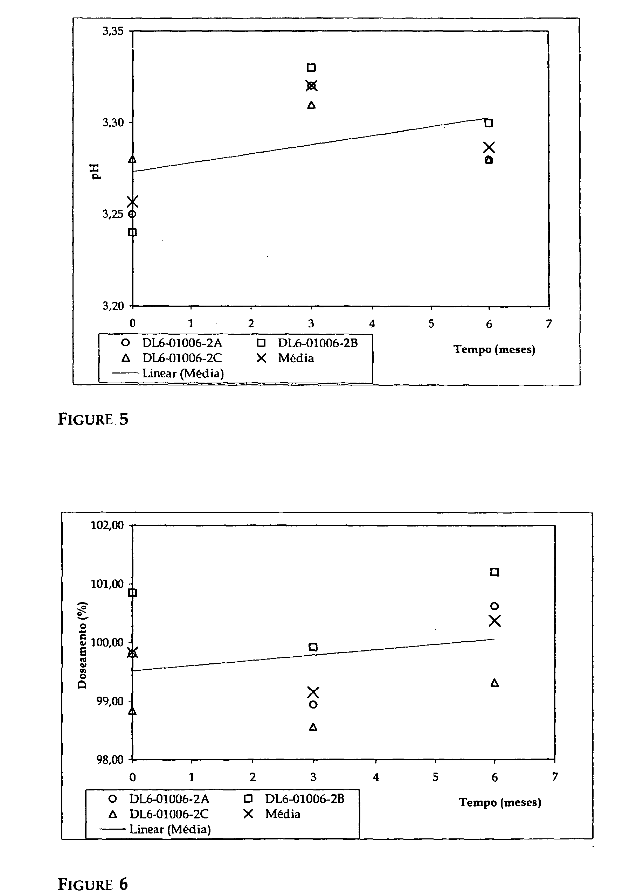

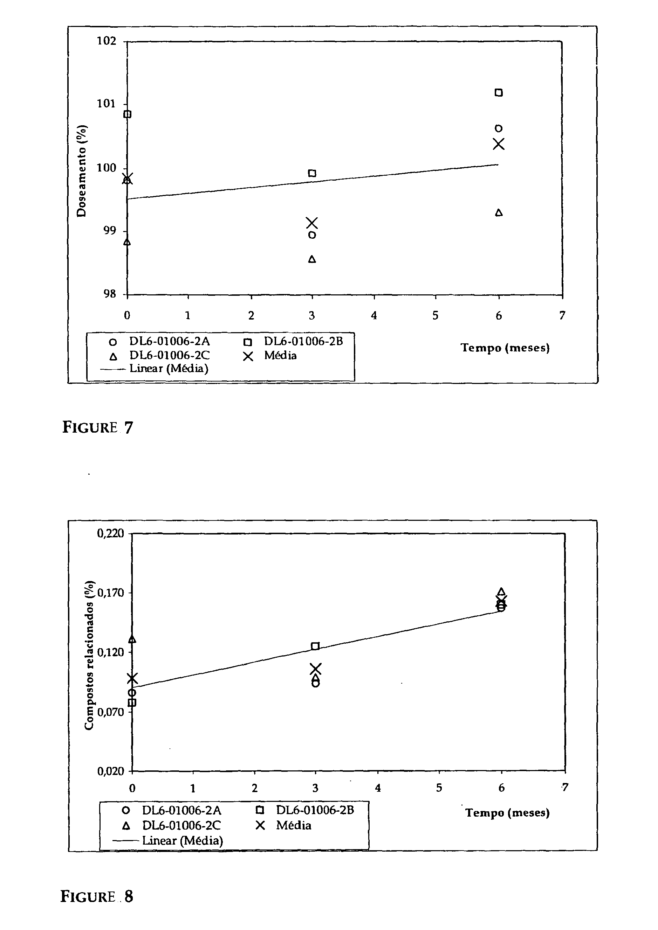

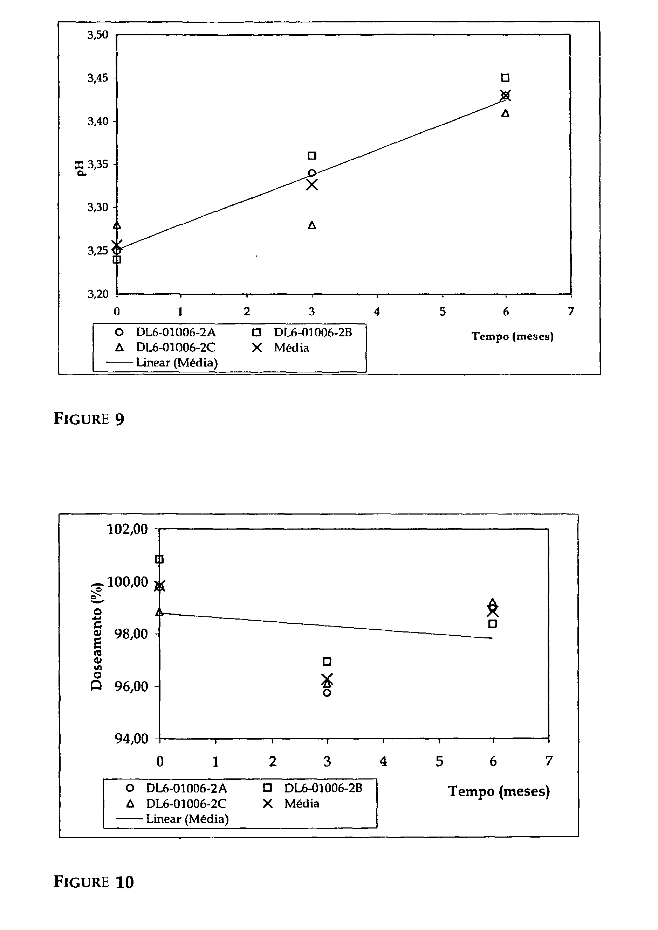

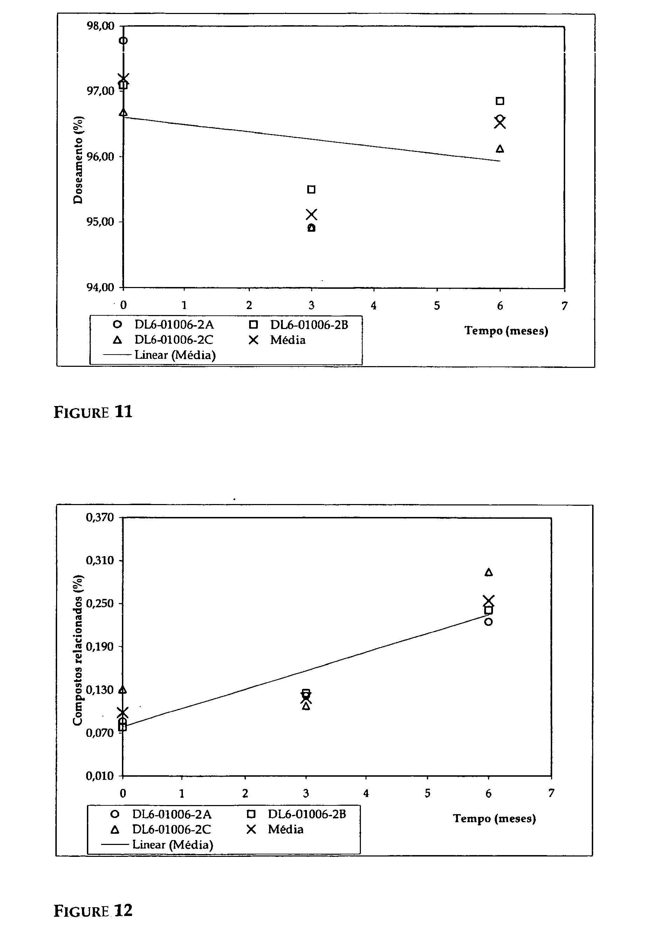

Figures 1 to 12 confirm the high stability of the developed formulation. The product kept its characteristics with respect to the studied parameters, even when the paste was stored at 40°C/75%RH during 6 months. The total content determined for the related compounds was less than 0.3% (w/w).

## Claims

1. Pharmaceutical composition **characterized in that** it consists in a diltiazem hydrochloride paste comprising in its composition only oily excipients.

2. Pharmaceutical composition according to claim 1, **characterized in that** it comprises 0.5% to 10% of diltiazem hydrochloride, relatively to the formulation total mass.

3. Pharmaceutical composition according to claim 1, **characterized in that** it comprises a combination of one or more emollients as oily excipients, one or more tensioactive agents promoters of the cutaneous absorption, and one or more thickeners.

4. Pharmaceutical composition according to claim 1, **characterized in that** it comprises one or more in oily excipients soluble preservatives.

5. Pharmaceutical composition according to claim 3, **characterized in that** coco-caprilate/caprate and liquid paraffin are used as emollients, ketostearyl alcohol is used as thickener, and polysorbate 40 is used as a tensioactive agent promoter of the cutaneous absorption.

6. Pharmaceutical composition according to claim 4, **characterized in that** it comprises benzoic acid, chlorbutanol or phenylethyl alcohol as preservatives.

7. Pharmaceutical composition according to claim 1, **characterized in that** it has a pH value between 2.5 and 5.

8. Pharmaceutical composition according to claim 1, **characterized in that** it has a viscosity value between 100 mPas and 1500 mPas, at 25°C.

9. Process for preparing a pharmaceutical composition according to claim 1, **characterized in that** the preservative is solubilized in one of the oily excipients, the oily excipients are melted, and for the fact that the diltiazem hydrochloride is added to the oily excipients paste at a temperature in the range of 45°C to 55°C, so that a homogeneous dispersion of the active substance is obtained.

10. Use of the pharmaceutical composition according to claims 1 to 8, **characterized in that** it is for the manufacture of a medicament for application to the skin and mucosa.

11. Use of the pharmaceutical composition according to claim 10, **characterized in that** the mentioned skin and mucosa is the anorectal mucosa.

## Patentansprüche

1. Pharmazeutische Zusammensetzung, **dadurch gekennzeichnet, daß** sie aus einer diltiazemchlorhydrathaltigend Paste, die in seiner Zusammensetzung einen öligen Bestandteil umfaßt, besteht.

2. Pharmazeutische Zusammensetzung nach Anspruch 1, **dadurch gekennzeichnet, daß** sie 0,5% bis 10% des Diltiazemchlorhydrats enthält, bezogen der ganze Masse der Formulierung.

3. Pharmazeutische Zusammensetzung nach Anspruch 1, **dadurch gekennzeichnet, daß** sie eine Vereinigung eines oder mehrer erweichenden Mitteln als öligen Bestandteile, eines oder mehrer oberflächenaktiven Stoffe, die die Absorption durch die Haut fördern, und eines ou mehrer Verdickungsmitteln umfaßt.

4. Pharmazeutische Zusammensetzung nach Anspruch 1, **dadurch gekennzeichnet, daß** sie einen oder mehr in öligen Bestandteile löslich Konservierungsmitteln umfaßt.

5. Pharmazeutische Zusammensetzung nach Anspruch 3, **dadurch gekennzeichnet, daß** als erweichenden Mitteln Kokos Kapryl-/Kaprinsäureestern und flüssige Paraffin verwendet werden, daß als Verdickungsmittel Ketostearylalkohol verwendet wird, und daß als oberflächenaktiven Stoff die die Absorption durch die Haut fördert Polyssorbat 40 verwendet wird.

6. Pharmazeutische Zusammensetzung nach Anspruch 4, **dadurch gekennzeichnet, daß** sie Benzoesäure, Chlorbutanol oder Phenylethylalkohol als Konservierungsmitteln umfaßt.

7. Pharmazeutische Zusammensetzung nach Anspruch 1, **dadurch gekennzeichnet, daß** sie einen pH-Wert zwischen 2,5 e 5 hat.

8. Pharmazeutische Zusammensetzung nach Anspruch 1, **dadurch gekennzeichnet, daß** sie eine Viskosität zwischen 100 mPas und 1500 mPas bei 25°C hat.

9. Verfahren zur Herstellung einer pharmazeutische Zusammensetzung nach Anspruch 1, **dadurch gekennzeichnet, daß** der Konservierungsmittel in einer der öligen Bestandteile gelöst wird, daß die Bestandteile geschmolzen werden und daß das Diltiazemchlorhydrat der Paste der öligen Bestandteile, bei einer Temperatur zwischen 45°C und 55°C, zugegeben wird, so daß eine homogenes Dispersion des Wirkstoffs erhalten wird.

10. Verwendung der pharmazeutischen Zusammensetzung nach Ansprüche 1 bis 8, **dadurch gekennzeichnet, daß** sie für die Herstellung eines Arzneimittel zur Verwendung auf der Haut und Schleimhäute verwendet wird.

11. Verwendung der pharmazeutischen Zusammensetzung nach Anspruch 10, **dadurch gekennzeichnet, daß** die oben gennanten Haut und Schleimhaut die Schleimhaut des Afters und des Mastdarms sind.

## Revendications

1. Composition pharmaceutique, **caractérisée en ce qu'**elle est composée d'une pâte de chlorhydrate de diltiazem, ne comprenant pas dans sa composition que des excipients huileux.

2. Composition pharmaceutique selon la revendication 1, **caractérisée en ce qu'**elle contient 0,5% à 10% du chlorhydrate de diltiazem, par rapport à la masse totale de la formulation.

3. Composition pharmaceutique selon la revendication 1, **caractérisée en ce qu'**elle contient une combinaison de un ou plusieurs émollients, en tant que des excipients huileux, une ou plusieures substances tensioactives promotrices de l'absorption par la peau, et une ou plusieures substances épaississantes.

4. Composition pharmaceutique selon la revendication 1, **caractérisée en ce qu'**elle comprend un ou plusieurs agents de conservation solubles dans des excipients huileux.

5. Composition pharmaceutique selon la revendication 3, **caractérisée en ce que** des caprylates/caprates de coco et de la paraffine liquide sont utilisés comme émollients, **en ce que** l'alcool cètostéarylique est utilisé comme épaississant, et **en ce que** le polyssorbat 40 est utilisé comme agent tensioactif promoteur de l'absorption par la peau.

6. Composition pharmaceutique selon la revendication 4, **caractérisée en ce qu'**elle comprend de l'acide benzoïque, du chlorobutanol ou de l'alcool phenyléthylique comme des agents de conservation.

7. Composition pharmaceutique selon la revendication 1, **caractérisée en ce qu'**elle a un valeur de pH compris entre 2,5 e 5.

8. Composition pharmaceutique selon la revendication 1, **caractérisée en ce qu'**elle a un valeur de viscosité entre 100 mPas et 1500 mPas, à 25°C.

9. Procédé pour la préparation d'une composition pharmaceutique selon la revendication 1, **caractérisé en ce que** l'agent de conservation est solubilisé dans un des excipients huileux, **en ce que** les excipients sont fondus, e **en ce que** le chlorhydrate de diltiazem est ajouté à la pâte des excipients huileux à une température dans l'intervalle de 45°C à 55°C, à fin qu'une dispersion homogène de la substance active soit obtenue.

10. Utilisation de la composition pharmaceutique selon les revendications 1 à 8, **caractérisée en ce qu'**elle est utilisée pour la préparation d'un médicament pour l'application sur la peau et les muqueuses.

11. Utilisation de la composition pharmaceutique selon la revendication 10, **caractérisée en ce que** la peau et la muqueuse suscités sont la muqueuse anale-rectale.
